Europäisches Patentamt

European Patent Office

Office européen des brevets

(11) Publication number: **0 375 649**
**A2**

# EUROPEAN PATENT APPLICATION

(21) Application number: **89870210.5**

(22) Date of filing: **21.12.89**

(51) Int. Cl.5: **A61K 9/26, A61K 9/22,**
**A61K 37/26, A61K 37/02**

(30) Priority: **22.12.88 US 288065**

(43) Date of publication of application:
**27.06.90 Bulletin 90/26**

(84) Designated Contracting States:
**AT BE CH DE ES FR GB GR IT LI LU NL SE**

(71) Applicant: **MONSANTO COMPANY**
**800 North Lindbergh Boulevard**
**St. Louis Missouri 63167(US)**

(72) Inventor: **Greenley, Robert Ziolkowski**
**655 Oak Valley Drive**
**Frontenac Missouri 63131(US)**

(74) Representative: **Lunt, John Cooper et al**
**Monsanto Services International S.A. Patent**
**Department Avenue de Tervuren 270/272**
**L.Box 21**
**B-1150 Brussels(BE)**

(54) **Swelling-controlled oral delivery system for drugs.**

(57) Swelling-controlled drug delivery system for enteral administration of a therapeutic agent includes a therapeutic agent imbibed in a polymeric material which swells minimally in a gastric environment with no significant loss of said therapeutic agent and swells extensively while remaining insoluble in an environment having a pH value greater than that of the gastric environment with release of an effective amount of the therapeutic agent.

EP 0 375 649 A2

# SWELLING-CONTROLLED ORAL DELIVERY SYSTEM FOR DRUGS

## BACKGROUND OF THE INVENTION

### 1. Field of the Invention

The present invention relates to controlled release oral delivery systems for drugs such as therapeutic agents and pharmaceuticals. More particularly, the present invention relates to polymeric swelling-controlled drug release formulations for delivery of drugs to a point beyond the stomach such as to the small intestine or colon. The subject formulations are particularly suitable for delivery of therapeutic agents which are normally destroyed by gastric juices in the gastrointestinal tract, or which cause gastric irritation. The subject formulations comprise a cross-linked polyacid resin having a selected drug imbibed or entrapped therein and are characterized in that they swell minimally in a gastric environment with no significant loss of drug and swell extensively, while remaining insoluble, in an environment having a pH value greater than that of the gastric environment, such as in an intestinal environment, to release an effective amount of the drug therein.

### 2. Description of Related Art

Enteral or oral administration of drugs is the most common method of administration, generally because it is the safest, most convenient and most economical. However, not all drugs are suitable for enteral administration. For example, drugs that are destroyed by gastric juices, such as insulin, or that cause gastric irritation, such as aspirin, are administered, respectively, by parenteral injection or in dosage forms with a coating that controls dissolution in the acidic gastric contents.

Controlled release of therapeutic agents is dependent upon the dissolution rate of the coating which is sometimes irregular because of variations in gastrointestinal pH, gastric emptying and other physiological factors. Moreover, absorption from the gastrointestinal tract is often incomplete and erratic.

Parenteral injection of drugs, particularly on a day-to-day basis, also has disadvantages associated therewith. Unfavorable reactions are prone to occur due to high concentrations of drugs which are attained rapidly in both plasma and tissues. Furthermore, repeated intravenous injections are dependent upon the ability to maintain a patent vein.

Therefore, it is preferable, when possible, to administer drugs enterally. To this end, many attempts have been made at developing drug delivery systems for enteral administration of drugs which, historically, have been administered parenterally or are known to cause gastric irritation.

One such attempt is described in U.S. Patent No. 4,663,308 wherein azo polymers are utilized to provide delivery of drugs to or through the mucosa of the large intestine. The drug is coated with a polymer of ethylenically unsaturated monomers cross-linked by a divinylazobenzene composition. The azo bonds are said to be stable to digestive juices in the mouth, stomach or small intestine and subject to degradation, by azo reductases existing in the large intestine. Once the polymer coating begins to degrade, the drug is released into the large intestine where, depending upon the specific therapeutic action of the drug, it either is absorbed through the mucosa of the large intestine or begins to act at that location.

Other attempts include those described in U.S. Patent Nos. 4,575,539 and 4,423,099 and references cited therein which utilize polymeric materials to form hydrogels (pH-independent, water-swellable, cross-linked polymers based primarily on 2-hydroxyethylmethacrylate). However, hydrogels typically swell under gastric conditions and thereby release a significant amount of drug in the stomach.

Swelling-controlled release systems are relatively new devices of the controlled release family of delivery devices for applications in pharmaceutical technology. To date, emphasis has been placed on swelling-controlled systems for release of a drug at a constant rate over a period of time (zero-order systems). Such systems are discussed by R. W. Korsmeyer and N. A. Peppas in Controlled Release Delivery Systems, Edited by T. J. Roseman and S. Z. Mansdorf, Chapter 4, pp. 77-90, Marcel Dekker, Inc. Publishers (1983). Specific systems are noted at pp. 87-89. Although no successful pharmaceutical formulation of this type "is yet known in the literature", it is suggested that possible modifications of the solubility of the polymer may lead to a desirable formulation.

## BRIEF SUMMARY OF THE INVENTION

The present invention resides in the discovery that cross-linked polyacid resins having swelling characteristics which are pH-dependent are particularly suited for formulating swelling-controlled systems for site-specific delivery of drugs to those portions of the gastrointestinal tract which are beyond the stomach and at higher pH values. The subject invention is particularly useful for delivery of relatively high molecular weight protein and polypeptide drugs or therapeutic agents through the stomach and to the small intestine.

The present polymeric formulations include cross-linked polyacid resins which have a selected drug imbibed or entrapped therein. As utilized herein, thereapeutic agent, drug and pharmaceutical are synonomous. These formulations manifest minimal swelling in a gastric environment, i.e., the amount of therapeutic agent lost due to release by swelling is insignificant, typically less than about 25% by weight, at a pH of from about 1 to about 6. Furthermore, once past the stomach and in an environment having a higher pH value, i.e., at a pH of from about 6 or greater, such formulations manifest extensive swelling, i.e., to an extent sufficient to release an effective amount of a therapeutic agent therein, for example, into the small intestine, where, depending upon the specific therapeutic action of the agent, it either is absorbed through the mucosa of the small intestine or begins to act therein. It is postulated that swelling of the present formulation in environments at pH values greater than about 6 is due to polyanion formation therein. For example, it is postulated that swelling of the present formulation in the small intestine is due to polyanion formation of the polyacid with bicarbonates secreted from the pancreas.

The subject formulations are particularly effective where the therapeutic agent is a relatively high molecular weight protein or polypeptide, for example, insulin. Although "conventional wisdom" teaches that peptides and proteins cannot readily pass through the wall of the intestine (For example, see Oral Delivery of Polypeptides: Identifying and Overcoming the Rate Limiting Mechanisms of Degradation and Transport, Proc. Intern. Sypm. Control. Rel. Bioact. Mater. 15, No. 39, pp. 60-61 (1988), Controlled Release Society, Inc.), it is believed that such observation is derived from the improbability of an orally delivered peptide or protein being delivered to the intestinal wall without first being denatured or degraded by gastric pepsin, or by one or more of various proteases, encountered in the duodenal portion of the small intestine. Once a protein is fragmented by these enzymes, these fragments will then be further degraded by peptidases which are in high concentration in the cellular wall of the small intestine. however, the present polymeric swelling-controlled oral delivery system protects such polypeptides while moving through the stomach and duodenum so that degradation by gastric pepsin or a protease does not readily occur.

## DETAILED DESCRIPTION

The polymeric swelling-controlled drug delivery systems of the present invention comprise cross-linked insoluble polyacid resins having a therapeutic agent imbibed therein. These formulations are prepared by imbibing a solution of the therapeutic agent into the cross-linked polyacid resin with subsequent removal of solvent.

Suitable polyacid resins include resins of polycarboxylic acids such as homopolymers and copolymers comprising carboxylic acid monomers having up to about four carbon atoms per carboxylic acid radical, preferably up to about three carbon atoms per carboxylic acid radical. Examples of such monomers include substituted and unsubstituted acrylic acid, methacrylic acid, fumaric acid, maleic acid, itaconic acid and crotonic acid. Suitable acid monomers also include substituted and unsubstituted sulfonic acid monomers having characteristics similar to those of the carboxylic acid monomers. Examples of such sulfonic acid monomers include vinyl sulfonic acid and styrene sulfonic acid. Preferred monomers are acrylic acid and methacrylic acid. Such monomers can be co-polymerized with each other or with alpha-olefins such as ethylene. Examples of such suitable polyacid resins, include poly(acrylic acid), poly(methacrylic acid), poly-(vinyl-sulfonic acid), poly(p-styrenesulfonic acid), poly(styrene-co-maleic acid), poly(vinyl methyl ether-co-maleic acid), poly(acrylic acid-co-maleic acid), poly(ethylene-co-maleic acid), poly(acrylic-co-methacrylic) and the like. Preferred polycarboxylic acids include poly(acrylic acid), poly(methacrylic acid), poly(vinyl methyl ether-co-maleic acid), poly(ethyleneco-maleic acid) and poly(acrylic-co-methacrylic). Particularly preferred resins are poly(acrylic acid) and poly(methacrylic acid).

Preparation of such suitable polyacids is accomplished by combining monomer(s), a cross-linking agent and a polymerization initiator in a substantially salt-free aqueous solution, and stirring and heating the solution, preferably under a positive nitrogen pressure sufficient to maintain a nitrogen atmosphere, until polymerization is substantially complete. After cooling, the resulting gelatinous slurry is poured into an appropriate container and the solvent is removed, such as in a vacuum oven. Preferably, the resultant dry resin is then ground in a mill and the portion passing through a 40 mesh screen (<425$\mu$m) is utilized for subsequent drug loading.

Suitable cross-linking agents include monomers containing at least two vinyl groups. Examples of such cross-linking agents include butylene diacry late, ethylene di(meth)acrylate, divinyl benzene, ethylene glycol di(meth)acrylate, di-, tri- and tetraethylene glycol di(meth)acrylate, methylene bisacrylamide, as well as other conventional cross-linking agents which will form a cross-linked polymer with the polyacid.

Suitable polymerization initiators include water soluble peroxy and azo polymerization initiator compounds. Examples of water soluble peroxy compounds are sodium persulfate and potassium persulfate. Examples of water soluble azo polymerization initiators are 2,2'-azobis (N,N-dimethyleneisobutyramide), 4,4'-azobis (4-cyanopentanoic acid), 2,2'-azobis (2-amidino propane) dihydrochloride. A preferred initiator is potassium persulfate.

The cross-linking agent is employed in an amount which will render the polyacid completely insoluble yet not interfere with the swelling characteristics. Suitable amounts are employed in a weight ratio, of cross-linking agent to carboxylic acid monomer, of within the range of about 1:1 to about .001:1, and a weight ratio of cross-linking agent to polymerization initiator of within the range of from about 0.001:1 to about 0.005:1. Preferably the amount of cross-linking agent utilized does not contribute less than about 0.5 mol % nor more than about 20 mol % of the total monomer mixture. At levels higher than about 20 mol %, the amount of cross-linking affects and sometimes impedes absorption of the therapeutic agent into the polymer. It is well within the skill of one in the art to determine which therapeutic agents are adapted to be absorbed into a polyacid wherein more than about 20 mol % is cross-linked. At levels lower than about 0.5 mol % the therapeutic agent is more likely to leach out under gastric conditions. It is well within the skill of one in the art to determine which therapeutic agents are adapted to be utilized with polyacids wherein less than about 0.5 mol % of the polyacid is cross-linked.

The polymerizations are carried out according to conventional methods except that they are conducted in salt-free solutions. Conventional methods, such as disclosed in U.S. Patent No. 3,202,577, utilize a saturated salt solution, such as a saturated MgSO$_4$ solution. However, polymers produced according to such methods swell to a much lesser extent in both gastric and intestinal fluids and, most importantly, significant amounts of therapeutic agent leach from such resins under gastric conditions. Thus another aspect of the present invention resides in the discovery that when the polymerizations of polycarboxylic acids are carried out in the substantial absence of MgSO$_4$ and other salts, such polyacids swell only minimally when exposed to gastric conditions without any significant leaching of the therapeutic agent imbibed therein, yet, when exposed to conditions of higher pH, such as when exposed to intestinal conditions, swell to an extent sufficient to release a therapeutic amount of the agent. Such resins are herein referred to as "salt-free" resins.

Suitable agents include those which are adapted to be absorbed by the polyacids described above and which are adapted to be released therefrom, upon swelling, in an amount sufficient to effect therapeutic action. Preferred therapeutic agents are peptides, pseudopolypeptides. polypeptides and proteins such as, for example, insulin, glucagon, parathyroid and pituitary hormones, calcitonin, vasopressin, renin, prolactin, thyroid stimulating hormone, corticotrophin, follicle stimulating hormone, luteinising hormone, chorionic gonadotrophin, somatotropins (growth hormones) and the like as well as 5-aminosalicylic acid (5-ASA). A particularly preferred therapeutic agent is insulin. Another preferred agent is 5-ASA. The amount of the agent incorporated into the polymer can vary over a wide range depending on the activity of the agent, the desired effect and other factors. Preferably, the agent is incorporated in an amount ranging from about 0.1 to about 200 mg agent per gram of polymer, such as from about 20 to about 150.

The therapeutic agent may be incorporated into the polymer by any technique which will cause the therapeutic agent to be absorbed into the polymer as opposed to absorbed onto the surface thereof (which would allow the agent to be released in the stomach). A preferred technique is to stir the polymer in a non-polar non-solvent and add an aqueous solution of the therapeutic agent dropwise over a time period of approximately one minute. Stirring is continued until it appears that the polymer is no longer swelling, typically from about 30 minutes to about two hours. The polymer/therapeutic agent matrix is isolated by decanting the organic layer and, where the therapeutic agent solvent is water, lyophilizing the solid residue. If the therapeutic agent solvent is non-aqueous, stirring is conducted in air and the solvent may be removed under vacuum in an oven with or without heat depending on the sensitivity of the drug. The dried solid is then preferably compacted into tablets utilizing a Parr press or other conventional tableting equipment known to those skilled in the art. Of course, other model of enteral delivery may be utilized and any of the

4

known processing steps for forming such delivery systems may also be utilized.

The amount of therapeutic agent/polyacid utilized will depend on the particular delivery system, the desired therapeutic effect, characteristics of both the polyacid and therapeutic agent, as well as other factors. Thus, optimal amounts of the components and of the formulations of the present invention can be easily determined by one skilled in the art.

When the polymer entrapped therapeutic agent is enterally administered, it passes through the stomach, with minimal swelling of the polymer and with minimal loss of therapeutic agent, and into the lower portions of the gastrointestinal tract, such as to the intestine, where, due to the presence of bicarbonates which are produced in the pancreas, the pH values are greater than the pH value in the stomach. Upon exposure to the higher pH values, the polymer begins to swell and thereby releases an effective amount of the therapeutic agent.

The following examples are non-limiting illustrative embodiments of the present formulations and their method of preparation. Variations thereof will be obvious to those skilled in the art.

## EXAMPLE 1

This example illustrates release of a therapeutic agent from a polymer formulation of the present invention under simulated gastric and intestinal environments.

An aqueous solution containing five wt.% monomer, from about 0.5 to about 10 mol% (based on the monomer), triethylene glycol di(meth)acrylate and one wt.% (based on the monomer) potassium persulfate were stirred under a positive nitrogen pressure at 55°C for three hours and 65°C for two additional hours. After cooling, the gelatinous slurry was poured into an appropriate container and the water removed in a vacuum oven at 75°C with a nitrogen bleed. The resin was then ground in a mill and the portion passing through a 40 mesh screen i.e., <425μm, was retained for drug loading according to the following procedure.

One gram of the resin was placed in a 50 ml Erlenmeyer flask, covered with 25 ml hexane and the slurry stirred magnetically. Five ml of an aqueous solution of the therapeautic agent was added dropwise over a period of about one minute. Stirring was continued for about one hour. The resin/therapeutic agent mixture was isolated by decanting the organic layer and lyophilizing the solid residue. The dried solid was then compacted into tablets utilizing a Parr press.

A spectroscopic working curve was then developed for varying concentrations of therapeutic agents in water.

Two 0.5 gram resin/drug tablets were placed in an Erlenmeyer flask and magnetically stirred in 50 ml of the synthetic gastric fluid described below (pH~1). After one hour, the flask contents were centrifuged and the supernate decanted and assayed for its drug content.

The solid was washed into a second flask with 50 ml of the synthetic intestinal fluid (pH~7) and 70 ml of the isotonic bicarbonate solution described below (which is sufficient to neutralize the acid function of the resin resulting in a slurry pH of about 7 to 8). After stirring two hours, the slurry was centrifuged and the supernate was assayed for released drug by UV at 254nm.

Results utilizing various polymers, various amounts of cross-linking agent and various polypeptide therapeutic agents are illustrated in Table 1.

TABLE 1

| % EXTRACTION OF IMBIBED AGENTS FROM POLYACID RESINS UNDER SYNTHETIC GASTRIC (1 HR) FOLLOWED BY INTESTINAL (2 HRS.) CONDITIONS | | | | | | | | |
|---|---|---|---|---|---|---|---|---|
| | Human Serum Albumin | | Insulin | | Tyr-Ala Dipeptide | | 5-Amino-Salicylic Acid | |
| | (50mg/g) | | (50mg/g) | | (40mg/g) | | (40mg/g) | |
| PROTECTING RESIN | gast | int | gast | int | gast | int | gast | int |
| Acrylic Acid | | | | | | | | |
| 2% cross-linked | 0 | 64 | 0 | 55 | 79 | 10 | 77 | 24 |
| 5% cross-linked | | | 10 | 78 | | | | |
| 10% cross-linked | | | 5 | 73 | | | 87 | 13 |
| Methacrylic Acid | | | | | | | | |
| 3% cross-linked | 0 | 75 | 4 | 28 | 10 | 41 | 12 | 64 |
| 5% cross-linked | | | 1 | 43 | | | | |
| 10% cross-linked | | | 2 | 31 | | | 69 | 31 |
| Ethylene/Maleic Anhydride Copolymer | | | | | | | | |
| 5% cross-linked | 0 | 96 | - | 100 | 92 | 20 | 78 | 32 |
| Methyl Vinyl Ether/Maleic Anhydride Copolymer | | | | | | | | |
| 5% cross-linked | 0 | 17 | 2 | 10 | 31 | 36 | 49 | 41 |

## EXAMPLE 2

This example demonstrates the difference between "salt polymerized" resins and those prepared in the substantial absence of salt, i.e., salt-free resins, according to the teachings of the present invention.

Poly(acrylic acid) and poly(methacrylic acid) resins (5 mol % cross-linking) were prepared as in Example 1 with the polymerizations being carried out in a saturated $MgSO_4$ solution. These resins were imbibed with insulin and compared with insulin-imbibed resins obtained according to the procedure of Example 1 in synthetic gastric and intestinal fluids. The results, which are presented in Table 2, demonstrate that "salt polymerized" resins not only swell to a much lesser extent, but also that significant amounts of insulin are leachable therefrom.

Swelling was determined by the following procedure. One gram of the resin was placed in a graduated cylinder and shaken with 25ml of a synthetic gastric fluid (2g NaCl, 7 ml concentrated HCl, diluted to one liter with water). A second one gram portion was shaken in a graduated cylinder with 50ml of synthetic intestinal fluid (6.8g NaCl, 5.0g $NaH_2PO_4.H_2O$, 0.8g $Na_2HPO_4.7H_2O$, diluted to one liter with water) and 70 ml of an isotonic bicarbonate solution (13.3g $NaHCO_3$ diluted to one liter with water). Both test cylinders were allowed to stand overnight and the volume of the settled resin was recorded.

The amount of insulin leached after two hours in synthetic gastric fluid was determined by HPLC according to the following procedure.

## INSULIN HPLC SCHEME

Column: Whatman ODS CSK Guard Colum

Whatman Protesil 300 Octyl, 10um
Solvent A: 0.1% TFA in water
Solvent B: 0.1% TFA in acetonitrile
Gradient: 0-3.6 min, hold 15%B
3.6-4.6 min, linear, 15-32%B
4.6-14 min, linear, 32-41.4%B
14-15 min, linear, 41.4-90%B
15-18 min, hold 90%B
18-19 min, linear, 90-15%B
19-27 min, hold 15%B
Flow Rate: 0-15 min, 1.5 ml/min
15-24 min, 2.5 ml/min
24-27 min, 1.5 ml/min
Detection: UV, 254 nm
Sample: 50 ul

TABLE 2

| | Swelling, ml | | |
|---|---|---|---|
| Resin | Gastric | Intestinal | Gastric Environment Leached Insulin % |
| Acrylic | | | |
| Acid($H_2O$) | 6 | 30 | 12 |
| Acid($MgSO_4$) | 4 | 10 | 44 |
| Methacrylic | | | |
| Acid($H_2O$) | 7 | 20 | 24 |
| Acid($MgSO_4$) | 4 | 7.5 | 64 |

EXAMPLE 3

This example illustrates that release of therapeutic agents from hydrogel formulations is not dependent on pH and that such formulations do not inhibit release of such agent under gastric conditions.

A hydroxyethyl methacrylate/acrylic acid hydrogel was prepared. Twenty grams of methacrylate ester and acrylic acid, in a 1:1 molar ratio, were stirred in 300 ml of water containing 0.15 g of potassium persulfate and about 5 mol % (based on the total molar amount of monomers) triethylene glycol diacrylate, and heated at 95° C for two hours under nitrogen. Water was then evaporated in an oven at 75° C under vacuum until the polymer was dry. The hydrogel was then ground in a mill and the portion passing through a 40 mesh screen was retained for drug loading.

Insulin was added to the hydrogel, according to the procedure set forth in Example 1 for formulating the polyacid resin/therapeutic agent of the present invention, in an amount to effect samples containing 50 mg of insulin per gram of hydrogel.

The samples were utilized in simulated gastric and intestinal environments according to the procedure set forth in Example 1 and assayed, using the HPLC procedure described in Example 2, to determine the amount of insulin released. Results are reported in Table 3.

TABLE 3

| Time(hrs.) | pH | Insulin Released(mg) | % Release |
|---|---|---|---|
| 0.08 | 1.45 | 24.2 | 48.4 |
| 0.33 | 1.34 | 37.1 | 74.2 |
| 0.67 | 1.36 | 39.0 | 78.0 |
| 1.00 | 1.37 | 40.0 | 80.1 |
| 1.33 | 1.36 | 40.1 | 80.3 |
| 1.67 | 1.37 | 40.1 | 80.3 |
| 2.00 | 1.39 | 39.2 | 78.5 |
| 0.08 | 7.75 | 22.3 | 44.6 |
| 0.50 | 7.46 | 30.4 | 60.9 |
| 1.00 | 7.80 | 32.0 | 64.1 |
| 2.00 | 7.64 | 35.3 | 70.6 |
| 3.00 | 7.61 | 37.5 | 75.1 |
| 4.00 | 7.65 | 38.0 | 76.1 |

## EXAMPLE 4

This example illustrates the effectiveness of the formulations of the present invention over a range of therapeutic agent loading.

Formulations were prepared according to the procedure set forth in Example 1 (with 5 mol % cross-linking) and assayed at various pH levels utilizing the HPLC procedure described in Example 2. Results are reported in Table 4.

TABLE 4

| Resin | Mg Insulin/Gram Resin | Time (hrs.) | pH | Insulin Released(%) |
|---|---|---|---|---|
| Poly (Methacrylic Acid) | 50 | 2 | 0.9 | 20.60 |
| | 50 | 2 | 2.8 | 0.02 |
| | 50 | 2 | 5.0 | 0.02 |
| | 50 | 2 | 7.6 | 35.52 |
| | 100 | 2 | 1.11 | 12.76 |
| | 100 | 2 | 4.71 | 1.16 |
| | 100 | 2 | 7.58 | 22.92 |
| Polyacrylic Acid | 50 | 2 | 1.23 | 16.33 |
| | 50 | 2 | 4.74 | 2.04 |
| | 50 | 2 | 6.86 | 90.44 |

## EXAMPLE 5

In this example, the efficary in diabetic rats is demonstrated for orally administered insulin imbibed in a polyanionic resin according to the teachings of the present invention.

Powdered resins containing 50 mg of insulin per gram of poly(acrylic acid) and poly(methacrylic acid) resin were prepared according to the procedures set forth in Example 1. A poly(methacrylic acid) containing 100 mg of insulin per gram of resin was also prepared utilizing the procedures of Example 1.

Rats were treated with 50 mg/kg of streptozotocin (STZ) which renders the rats diabetic by destroying the pancreatic islets which produce insulin. The urine of the rats was monitored with "glucose sticks" to assure that there was an increase in glucose output.

The rats were allowed to eat and drink freely (ad libidum) and were treated with 18 Units of bovine serum insulin in the polyacid resins twice a day. This was repeated daily for two weeks. Two hours after the last dose, the rats were sacrificed and their blood stored (refrigerated) for subsequent assay.

Group 1-4 (A-50) was treated with poly (acrylic acid) resin containing 50 mg insulin/g resin. Group 6-10 (M-100) was treated with poly(methacrylic acid) resin containing 100 mg insulin/g resin. Group 16-20 (Gel) was a positive control group treated with a compounded formulation of human insulin in gelatin (dissolves in the stomach). Group 21-25 was the neutral control and did not get STZ or insulin. Group 27-31 was the negative control and was rendered diabetic but received no insulin. The results show that in the case of the first formulation, 50 mg insulin in lg acrylic acid resin, there were 3/4 rats with serum glucose reductions and higher levels of serum insulin than found for the diabetic, no treatment group. A similar finding also resulted for 2/4 responding rats treated with the second formulation, 50 mg insulin in lg methacrylic acid resin. The third formulation, 100 mg insulin in lg methacrylic acid resin, was an ineffective treatment at the same insulin dose level. The rationale for this lack of activity is that the higher insulin level results in a more adherent powder that has been shown to have a slower rate of drug release in in vitro tests (Example 4).

## TABLE 5

|  | Rat Number | % Weight Gained | Glucose Serum Conc. mg/dL | Insulin Serum Conc. μU/ml |
|---|---|---|---|---|
| Group A-50 | 1 | 50 | 317 | 119 |
|  | 2 | 62 | 460 | 77 |
|  | 3 | 62 | 171 | 353 |
|  | 4 | 59 | 380 | 198 |
| Group M-50 | 6 | 55 | 502 | 128 |
|  | 7 | 66 | 181 | 133 |
|  | 9 | 46 | 476 | 95 |
|  | 10 | 63 | 203 | 170 |
| Group M-100 | 11 | 36 | 487 | 101 |
|  | 12 | 20 | 514 | 113 |
|  | 13 | 40 | 447 | 105 |
|  | 14 | 34 | 512 | 109 |
|  | 15 | 61 |  |  |
| Group Non-DIAB No-TREAT | 21 | 81 | 165 | 80 |
|  | 22 | 74 | 173 | -- |
|  | 23 | 82 | 178 | 59 |
|  | 24 | 81 | 239 | 49 |
|  | 25 | 71 | 161 | 73 |
| Group DIAB No-TREAT | 27 | 64 | 514 | 27 |
|  | 28 | 63 | -- | 79 |
|  | 29 | 57 | 538 | 89 |
|  | 30 | 59 | 498 | 39 |
|  | 31 | 64 | 433 | 39 |

## Claims

1. Swelling-controlled drug delivery system for enteral administration of a therapeutic agent comprising a therapeutic agent imbibed in a polyacid resin adapted to swell minimally in a gastric environment with no significant loss of said therapeutic agent, and swell extensively while remaining insoluble in an environment having a pH value greater than that of the gastric environment with release of an effective amount of said therapeutic agent.

2. Drug delivery system of Claim 1 wherein said polyacid resin is a salt-free resin.

3. Drug delivery system of Claim 2 wherein said polyacid resin is selected from the group consisting of poly(acrylic acid), poly(methacrylic acid), poly(vinyl sulfonic acid), poly(p-styrene sulfonic acid), poly-(styrene-co-maleic acid), poly(vinyl methyl ether-co-maleic acid), poly(acrylic acid-co-maleic acid), poly-(ethylene-co-maleic acid) and poly(acrylic acid-co-methacrylic acid).

4. Drug delivery system of Claim 3 wherein from about 0.5 to about 20 mol % of the resin is cross-linked.

5. Drug delivery system of Claim 4 wherein said therapeutic agent is adapted to be absorbed by said cross-linked polyacid resin and is adapted to be released therefrom, upon swelling of the resin, in an amount sufficient to effect therapeutic action.

6. Drug delivery system of Claim 5 wherein said therapeutic agent is a polypeptide.

7. Drug delivery system of Claim 6 wherein said polypeptide is selected from the group consisting of insulin, growth hormones (somatotropins) and 5-ASA.

8. Method of preparing a swelling-controlled drug delivery system of Claim 1 comprising stirring the polyacid resin in a non-polar non-solvent, adding an aqueous solution of the therapeutic agent dropwise thereto over a time period of about one minute, stirring until it appears that the polymer is no longer swelling, decanting the organic layer and removing any remaining solvent.

9. Swelling-controlled drug delivery system for enteral administration of a drug comprising a drug imbibed into a salt-free cross-linked polyacid resin, said polyacid being selected from the group consisting of polycarboxylic acids which include one or more acid monomers having up to about four carbon atoms per carboxylic acid radical and substituted and unsubstituted sulfonic acid monomers.

10. Delivery system of Claim 9 wherein said polyacid is selected from the group consisting of poly-(acrylic acid), poly(methacrylic acid), poly (vinyl sulfonic acid), poly(p-styrene sulfonic acid), poly(styrene-co-maleic acid), poly(vinyl methyl ether-co-maleic acid), poly(acrylic acid-co-maleic acid), poly(ethylene-co-maleic acid)and poly(acrylic acid-co-methacrylic acid).

11. Delivery system of Claim 9 wherein said polyacid is a polycarboxylic acid selected from the group consisting of poly(acrylic acid), poly(methacrylic acid), poly (ethylene-co-maleic acid),poly(acrylic acid-co-methacrylic acid) and poly(vinyl methyl ether-co-maleic acid).

12. Delivery system of Claim 9 wherein said polycarboxylic acid is selected from the group consisting of poly(acrylic acid) and poly(methacrylic acid).

13. Delivery system of Claim 12 wherein the drug is insulin.

14. Delivery system of Claim 12 wherein the drug is 5-ASA.

15. In a drug delivery system for enteral delivery of a drug, the improvement which comprises imbibing such drug in a cross-linked polyacid resin adapted to swell minimally in a gastric environment with no significant loss of the drug and swell extensively, without dissolving, in an environment having a pH value greater than that of the gastric environment with release of an effective amount of the drug.